# EUROPEAN PATENT APPLICATION

(11) **EP 4 534 680 A1**
(43) Date of publication of application: **09.04.2025**
(21) Application number: 23811840.0
(22) Date of filing: 24.05.2023
(51) Int. Cl.: C12N 15/86, A61K 31/7088, A61K 31/7105, A61K 31/713, A61K 35/12, A61K 38/16, A61K 48/00, A61P 35/00, C12N 5/078, C12N 5/10, C12N 15/113, C12N 15/12, C12N 15/13, C12N 15/62

(54) **POLYNUCLEOTIDE**

(30) Priority: 25.05.2022 JP 2022085408
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: TERAKURA, Seitaro, Nagoya-shi, Aichi 464-8601 (JP); ADACHI, Yoshitaka, Nagoya-shi, Aichi 464-8601 (JP); KIYOI, Hitoshi, Nagoya-shi, Aichi 464-8601 (JP)
(74) Representative: Hoffmann Eitle
(86) International application number: PCT/JP2023/019295
(87) International publication number: WO 2023/228968

(57) **Abstract**

To provide a technique for enhancing proliferation capability of CAR-expressing cells. To reduce expression of CUL5 gene.

## Description

### Technical Field

The present invention relates to a polynucleotide useful for producing cells expressing a chimeric antigen receptor, and the like.

### Background Art

A chimeric antigen receptor (hereinafter, also referred to as "CAR") binds to an antigen according to specificity of an antibody, and this can cause T cell division after target cell injury, cytokine release, and stimulation. It is possible to confer specificity to T cells by gene transfer, and preparation of cells is generally easier than when culturing and amplifying endogenous tumor-specific T cell receptor (TCR)-positive T cells. In addition, a treatment method using a CAR (CAR-T therapy) is excellent in that it exhibits a high cytotoxic activity as compared with an antibody therapy and that multiple treatments are not required for autonomously amplifying by antigen stimulation in one infusion. TCR gene transfer is more advantageous in that it is only treatable if the patient has a particular HLA (HLA-restricted) because it targets a complex of HLA and the peptide presented thereon, but the CAR is not HLA-restricted. That is, when the tumor cells are positive for the target antigen, all patients positive for the target antigen can be treated.

CD19-targeting chimeric antigen receptor gene transfer T cell (CAR-T cell) therapy has been approved as a drug in various countries around the world. Its clinical effect is high, and its clinical usefulness for various malignant tumors is expected (for example, Patent Literature 1).

### Citation List

### Patent Literature

PTL 1: Japanese Patent No. 5312721

### Summary of Invention

### Technical Problem

It is considered that a problem of recurrence and treatment resistance after CAR-T therapy is that the time during which CAR-T cells survive and play an active role in the patient's body is short. In order to solve this problem, it is desirable to increase proliferation capability (in particular, proliferation capability after contact with a target antigen) of CAR-T cells. It is also important to efficiently obtain CAR-T cells.

Therefore, an object of the present invention is to provide a technique for enhancing proliferation capability of CAR-expressing cells. Another object of the present invention is to provide a technique for efficiently obtaining CAR-expressing cells.

### Solution to Problem

As a result of intensive studies in view of the above objects, the present inventor has found that the proliferation capability of CAR-expressing cells is enhanced by reducing expression of CUL5 gene. As a result of further research based on this finding, the present inventor has completed the present invention. That is, the present invention includes the following embodiments.

Item 1. A polynucleotide containing an expression cassette of a CUL5 gene expression suppressing polynucleotide and an expression cassette of a chimeric antigen receptor.

Item 2. The polynucleotide according to item 1, in which the CUL5 gene expression suppressing polynucleotide is at least one selected from the group consisting of CUL5-specific siRNA, CUL5-specific miRNA, and a CUL5-specific antisense polynucleotide.

Item 3. The polynucleotide according to item 1, in which the CUL5 gene expression suppressing polynucleotide is CUL5-specific siRNA.

Item 4. The polynucleotide according to item 1, in which the polynucleotide is a vector.

Item 5. The polynucleotide according to item 1, in which the polynucleotide is a viral vector or a viral genome.

Item 6. The polynucleotide according to item 1, in which a target antigen of the chimeric antigen receptor is a cancer antigen.

Item 7. A cell containing the polynucleotide according to any of items 1 to 6.

Item 8. The cell according to item 7, in which expression of CUL5 gene is reduced by the CUL5 gene expression suppressing polynucleotide, and the cell expresses a chimeric antigen receptor.

Item 9. The cell according to item 8, in which the cell is a lymphocyte cell.

Item 10. A pharmaceutical composition containing the cell according to item 7.

Item 11. The pharmaceutical composition according to item 10, which is for use in a treatment, prevention, or improvement of cancer.

Item 12. A cell that is modified to reduce expression of CUL5 gene and expresses a chimeric antigen receptor.

Item 13. The cell according to item 12, in which the cell is a lymphocyte cell.

Item 14. A pharmaceutical composition containing the cell according to item 12 or 13.

Item 15. The pharmaceutical composition according to item 14, which is for use in a treatment, prevention, or improvement of cancer.

### Advantageous Effects of Invention

According to the present invention, it is possible to provide a technique for enhancing proliferation capability of CAR-expressing cells. Furthermore, according to a preferred embodiment of the present invention, it is possible to provide a technique for efficiently obtaining CAR-expressing cells.

### Brief Description of Drawings

Fig. 1 shows a schematic diagram of a CAR construct obtained in Test Example 1.
Fig. 2 shows an outline of GW-CRISPR screening (primary screening) of Test Example 2.
Fig. 3 shows results of secondary screening of Test Example 2. "day" indicates the number of elapsed days from first stimulation. The vertical axis is a value (%) obtained by subtracting the GFP-positive cell ratio (%) at day0 from the GFP-positive cell ratio (%) at day30. The gRNAs expressed in cells are shown in the horizontal axis.
Fig. 4 shows results of secondary screening of Test Example 2. The vertical axis is the GFP-positive cell ratio (%). In the horizontal axis, D indicates the number of elapsed days from the first stimulation. *** indicates P < 0.001 between groups.
Fig. 5 shows results of measuring the expression level of CUL5 by Western blotting when gRNA for CUL5 was expressed in the secondary screening of Test Example 2. The vertical axis represents the ratio of the expression level of CUL5 to the expression level of β-actin. The horizontal axis represents expressed gRNA. * indicates P < 0.05 between groups, and ** indicates P < 0.01 between groups.
Fig. 6 shows the degree of cell division measured in Test Example 3. The vertical axis represents fluorescence intensity. The lower the fluorescence intensity, the more the division is enhanced. In the horizontal axis, Ctrl indicates a case where control gRNA is expressed, and CUL5 KO indicates a case where CUL5 gRNA is expressed. * indicates P < 0.05 between groups.
Fig. 7 shows ratios of intracellular cytokine-positive cells measured in Test Example 3 (vertical axis). The stimulated cells are shown in the horizontal axis. Of the two columns of each of the stimulated cells, the left side shows a case where control gRNA is expressed, and the right side shows a case where CUL5 gRNA is expressed. * indicates P < 0.05 between groups.
Fig. 8 shows ratios of effector memory T cells (T_{EM}: CD45RA(-)CCR7(-)) in the left figure and central memory T cells (T_{CM}: CD45RA(-)CCR7(+)) in the right figure, measured in Test Example 4 (vertical axis). In the horizontal axis, Ctrl indicates a case where control gRNA is expressed, and CUL5 KO indicates a case where CUL5 gRNA is expressed. ** indicates P < 0.01 between groups.
Fig. 9 shows luminescence of tumor cells measured in Test Example 5 (vertical axis). The horizontal axis represents the number of days elapsed from tumor cell inoculation. Ctrl CAR indicates a case where CAR-T cells expressing control gRNA are administered, CUL5KO CAR indicates a case where CAR-T cells expressing CUL5 gRNA are administered, and tEGFR-T indicates a case where T cells expressing tEGFR are administered.
Fig. 10 shows survival rates measured in Test Example 5 (vertical axis). The horizontal axis represents the number of days elapsed from tumor cell inoculation. Ctrl CAR indicates a case where CAR-T cells expressing control gRNA are administered, CUL5KO CAR indicates a case where CAR-T cells expressing CUL5 gRNA are administered, and tEGFR-T indicates a case where T cells expressing tEGFR are administered. * indicates P < 0.05 between groups, *** indicates P < 0.001 between groups, and **** indicates P < 0.0001 between groups.
Fig. 11 shows a schematic diagram of two-in-one vector obtained in Test Example 6.
Fig. 12 shows a scheme of CAR-T cell adjustment using the two-in-one vector (before stimulation is started).
Fig. 13 shows the number of CAR-T cells measured in Test Example 7 (vertical axis). The left column shows the case of Method 1, and the right column shows the case of Method 2 (when the two-in-one vector is used). *** indicates P < 0.001 between groups.
Fig. 14 shows results of measuring the expression level of CUL5 by Western blotting in Test Example 7. Ctrl indicates a case where shRNA against GFP is expressed, and CUL5 KD indicates a case where shRNA against CUL5 is expressed.
Fig. 15 shows proliferation of CAR-T cells measured in Test Example 7. The vertical axis represents the number of CAR-T cells. The horizontal axis represents the number of days elapsed from the first stimulation. ** indicates P < 0.01 between groups.
Fig. 16 shows luminescence of tumor cells measured in Test Example 8 (vertical axis). The horizontal axis represents the number of days elapsed from tumor cell inoculation. Ctrl indicates a case where CAR-T cells expressing shRNA against GFP are administered, and CUL5KD indicates a case where CAR-T cells expressing shRNA against CUL5 are administered. Mock indicates a case where CAR-T cells are not administered.
Fig. 17 shows tumor volumes measured in Test Example 9 (vertical axis). The horizontal axis represents the number of days elapsed from tumor cell inoculation. shGFP CD19CAR-T indicates a case where shRNA against GFP is expressed, shCUL5 CD19CAR-T indicates a case where shRNA against CUL5 is expressed, and tEGFR-T indicates a case where T cells expressing tEGFR are administered. * indicates P < 0.05 between groups, and ** indicates P < 0.01 between groups.
Fig. 18 shows survival rates measured in Test Example 9 (vertical axis). The horizontal axis represents the number of days elapsed from tumor cell inoculation. shGFP CD19CAR-T indicates a case where shRNA against GFP is expressed, shCUL5 CD19CAR-T indicates a case where shRNA against CUL5 is expressed, and tEGFR-T indicates a case where T cells expressing tEGFR are administered. * indicates P < 0.05 between groups.
Fig. 19 shows photographed images of luminescence of Test Example 11. The number of days elapsed from tumor cell inoculation is shown on the left side of the photographs. The intracellular domain and shRNA are shown adopted above the photographs.
Fig. 20 shows tumor volumes measured in Test Example 11 (vertical axis). The horizontal axis represents the number of weeks elapsed from tumor cell inoculation. The adopted intracellular domain and shRNA are shown in the legend. Description of Embodiments

In the present specification, the expressions "containing" and "including" include the concepts of "containing", "including", "consisting essentially of", and "consisting only of".

### 1. Definitions

In the present specification, the expressions "containing" and "including" include the concepts of "containing", "including", "consisting essentially of", and "consisting only of".

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. "Methods for assessing the statistical significance of molecular sequence features by using general scoring schemes" Proc Natl Acad Sci USA. 87: 2264-2268 (1990), Karlin S, Altschul SF. "Applications and statistics for multiple high-scoring segments in molecular sequences." Proc Natl Acad Sci USA. 90: 5873-7 (1993)). A program called BLASTX based on this BLAST algorithm has been developed. The specific techniques of these analysis methods are known and can be found on the website of the National Center of Biotechnology Information (NCBI) (http://www.ncbi.nlm.nih.gov/). The "identity" of base sequences is also defined according to the above.

In the present specification, "conservative substitution" means the substitution of an amino acid residue with an amino acid residue having a similar side chain. For example, the substitution between amino acid residues having a basic side chain, such as lysine, arginine, or histidine, corresponds to a conservative substitution. In addition, the following substitutions between amino acid residues also correspond to conservative substitutions: the substitution between amino acid residues having an acidic side chain, such as aspartic acid and glutamic acid; the substitution between amino acid residues having an uncharged polar side chain, such as glycine, asparagine, glutamine, serine, threonine, tyrosine, or cysteine; the substitution between amino acid residues having a nonpolar side chain, such as alanine, valine, leucine, isoleucine, proline, phenylalanine, methionine, or tryptophan; the substitution between amino acid residues having a β-branched side chain, such as threonine, valine, or isoleucine; and the substitution between amino acid residues having an aromatic side chain, such as tyrosine, phenylalanine, tryptophan, or histidine.

In the present specification, "nucleic acid" and "polynucleotide" are not particularly limited, and include both natural and artificial ones. Specifically, in addition to DNA, RNA, and the like, the "nucleic acid" and "polynucleotide" may be known chemically modified ones as exemplified below. To prevent degradation by hydrolases, such as nucleases, the phosphate residue (phosphate) of each nucleotide can be substituted with, for example, a chemically modified phosphate residue, such as phosphorothioate (PS), methylphosphonate, or phosphorodithionate. Also, the hydroxyl group at position 2 of a sugar (ribose) of each ribonucleotide may be substituted with -OR (R represents, for example, CH3 (2'-O-Me), CH2CH2OCH3 (2'-O-MOE), CH2CH2NHC(NH)NH2, CH2CONHCH3, or CH2CH2CN). Additionally, the base moiety (pyrimidine, purine) may be chemically modified by, for example, introduction of a methyl group or a cationic functional group into position 5 of the pyrimidine base, or substitution of the carbonyl group at position 2 with thiocarbonyl. Furthermore, examples thereof include, but are not limited to, those in which the phosphoric acid moiety or hydroxyl moiety is modified with, for example, biotin, an amino group, a lower alkylamine group, an acetyl group, or the like. Moreover, BNA (LNA) or the like in which the conformation of the sugar moiety is fixed to N-type by crosslinking 2' oxygen and 4' carbon of the sugar moiety of the nucleotide can also be used.

In the present specification, "CDR" is an abbreviation for Complementarity Determining Region, and also referred to as complementarity determining region. The CDR is a region present in a variable region of an immunoglobulin or a T cell receptor, and is a region deeply involved in the specific binding of an antibody or T cell receptor to an antigen. The "light chain CDR" is a CDR present in a light chain variable region of an immunoglobulin, and the "heavy chain CDR" means a CDR present in a heavy chain variable region of an immunoglobulin. Also in the T cell receptor, there are α chain, β chain, γ chain, δ chain, and the like, and the same applies to the CDRs of these chains.

In the present specification, the "variable region" means a region containing CDR1 to CDR3 (hereinafter, simply referred to as "CDRs 1-3"). The arrangement order of these CDRs 1-3 is not particularly limited, but preferably means a region arranged in the order of CDR1, CDR2, and CDR3 in the direction from the N-terminal side to the C-terminal side in the reverse order either consecutively or via other amino acid sequences referred to as framework regions (FRs), which are described later. The "heavy chain variable region" is a region where the above-described heavy chain CDRs 1-3 are arranged in the immunoglobulin, and the "light chain variable region" is a region where the above-described light chain CDRs 1-3 are arranged in the immunoglobulin. Also in the T cell receptor, there are α chain, β chain, γ chain, δ chain, and the like, and the same applies to the CDRs of these chains.

The regions other than CDRs 1-3 of each variable region are referred to as framework regions (FRs) as described above. In particular, a region between the N-terminus and the CDR1 of the variable region is defined as FR1, a region between CDR1 and CDR2 is defined as FR2, a region between CDR2 and CDR3 is defined as FR3, and a region between CDR3 and the C-terminus of the variable region is defined as FR4.

### 2. Polynucleotides

In one embodiment, the present invention relates to a polynucleotide containing an expression cassette of a CUL5 gene expression suppressing polynucleotide and an expression cassette of a chimeric antigen receptor (sometimes referred to as the "polynucleotide of the present invention" in the present specification). The polynucleotide of the present invention will be described below.

The polynucleotide of the present invention contains an expression cassette of a CUL5 gene expression suppressing polynucleotide. The proliferation capability of CAR-expressing cells can be enhanced by preparing CAR-expressing cells using the polynucleotide of the present invention containing the expression cassette of a CUL5 gene expression suppressing polynucleotide. The CUL5 gene is a gene expressing Cullin-5 mRNA/protein. The Cullin-5 protein is a core component of multiple SCF-like ECS (Elongin-Cullin 2/5-SOCS-boxprotein) E3 ubiquitin-protein ligase complexes and mediates ubiquitination of target proteins and subsequent proteasomal degradation. The CUL5 gene expression suppression is to suppress the expression level of Cullin-5 mRNA/protein. The species derived from the CUL5 gene is not particularly limited, and examples thereof include various mammals, such as humans, monkeys, mice, rats, dogs, cats, rabbits, pigs, horses, cows, sheep, goats, and deer. The human CUL5 gene is a gene of NCBI gene ID: 8065.

The amino acid sequence of Cullin-5 protein and the base sequence of Cullin-5 mRNA derived from various species are known. Specific examples of the human Cullin-5 protein include a protein consisting of an amino acid sequence set forth in SEQ ID NO: 30 (NCBI Reference Sequence: NP_003469.2), and specific examples of the human Cullin-5 mRNA include an mRNA consisting of a base sequence set forth in SEQ ID NO: 31 (NCBI Reference Sequence: NM_003478.6). The Cullin-5 protein and Cullin-5 mRNA may also include splicing variants of the Cullin-5 protein and Cullin-5 mRNA described above.

The Cullin-5 protein expressed from the CUL5 gene whose expression is to be suppressed may have an amino acid mutation such as substitution, deletion, addition, or insertion as long as it forms an E3 ubiquitin-protein ligase complex having its inherent activity, i.e., ubiquitin E3 ligase activity. The mutation is preferably a substitution, more preferably a conservative substitution, from the viewpoint that the activity is less likely to be impaired.

The Cullin-5 mRNA expressed from the CUL5 gene whose expression is to be suppressed may also have a base mutation such as substitution, deletion, addition, or insertion as long as the protein translated from the mRNA forms an E3 ubiquitin-protein ligase complex having its inherent activity, i.e., ubiquitin E3 ligase activity. As the mutation, a mutation in which no amino acid substitution occurs or a mutation in which a conservative substitution of an amino acid occurs in a protein translated from the mRNA is preferable.

Preferable specific examples of the Cullin-5 protein expressed from the CUL5 gene whose expression is to be suppressed include at least one selected from the group consisting of a protein described in (A) below and a protein described in (B) below:
(A) a protein consisting of an amino acid sequence set forth in any of SEQ ID NO: 30; and
(B) a protein consisting of an amino acid sequence having equal to or greater than 85% identity to the amino acid sequence set forth in any of SEQ ID NO: 30 and forming an E3 ubiquitin-protein ligase complex having ubiquitin E3 ligase activity.

In (B) above, the identity is more preferably 90% or more, still more preferably 95% or more, and even still more preferably 98% or more.

An example of the protein described in (B) above is, for example,
(B') a protein consisting of an amino acid sequence having a substitution, deletion, addition, or insertion of one or more amino acids with respect to the amino acid sequence set forth in any of SEQ ID NO: 30 and forming an E3 ubiquitin-protein ligase complex having ubiquitin E3 ligase activity.

In (B') above, "a plurality of" means, for example, 2 to 50, preferably 2 to 20, more preferably 2 to 10, and even still more preferably 2 or 3.

Preferred specific examples of the Cullin-5 mRNA expressed from the CUL5 gene whose expression is to be suppressed include at least one selected from the group consisting of mRNA described in (C) below and mRNA described in (D) below:
(C) an mRNA consisting of a base sequence set forth in any of SEQ ID NO: 31; and
(D) an mRNA encoding a protein consisting of a base sequence having equal to or greater than 85% identity to the base sequence set forth in any of SEQ ID NO: 31 and forming an E3 ubiquitin-protein ligase complex having ubiquitin E3 ligase activity.

In (D) above, the identity is more preferably 90% or more, still more preferably 95% or more, and even still more preferably 98% or more.

An example of the mRNA described in (D) above is, for example,
(D') an mRNA encoding a protein consisting of a base sequence having a substitution, deletion, addition, or insertion of one or more amino acids with respect to the base sequence set forth in any of SEQ ID NO: 31 and forming an E3 ubiquitin-protein ligase complex having ubiquitin E3 ligase activity.

In (D') above, "a plurality of" means, for example, 2 to 200, preferably 2 to 100, more preferably 2 to 50, and even still more preferably 2 to 10.

The CUL5 gene expression suppressing polynucleotide is not particularly limited as long as it is a polynucleotide capable of suppressing the expression level of CUL5 protein, CUL5 mRNA, or the like, and examples thereof include CUL5-specific small interfering RNA (siRNA), CUL5-specific microRNA (miRNA), and CUL5-specific antisense polynucleotide.

The expression suppression means that the expression level of Cullin-5 protein is suppressed to, for example, 70% or less, 60% or less, 50% or less, 40% or less, 30% or less, 20% or less, or 10% or less, and also includes that the expression level is set to 0.

The CUL5-specific siRNA is not particularly limited as long as it is a double-stranded RNA molecule that specifically suppresses the expression of CUL5 gene. In one embodiment, the siRNA preferably has a length of, for example, 18 bases or more, 19 bases or more, 20 bases or more, or 21 bases or more. In addition, the siRNA preferably has a length of, for example, 25 bases or less, 24 bases or less, 23 bases or less, or 22 bases or less. It is assumed that the upper limit value and the lower limit value of the length of the siRNA described herein are arbitrarily combined. For example, the following combinations of the lengths are contemplated: a length in which the lower limit is 18 bases and the upper limit is 25 bases, 24 bases, 23 bases, or 22 bases; a length in which the lower limit is 19 bases and the upper limit is 25 bases, 24 bases, 23 bases, or 22 bases; a length in which the lower limit is 20 bases and the upper limit is 25 bases, 24 bases, 23 bases, or 22 bases; and a length in which the lower limit is 21 bases and the upper limit is 25 bases, 24 bases, 23 bases, or 22 bases.

The siRNA may be shRNA (small hairpin RNA). The shRNA can be designed so that a part thereof forms a stem loop structure. For example, when a sequence of a certain region is defined as sequence a and a complementary strand to the sequence a is defined as sequence b, the shRNA can be designed so that these sequences are present in one RNA strand in the order of the sequence a, a spacer, and the sequence b, and the whole length is 45 to 60 bases. The sequence a is a sequence of a partial region of the base sequence encoding CUL5 as a target, the target region is not particularly limited, and an arbitrary region can be used as a candidate. The length of the sequence a is 19 to 25 bases, and preferably 19 to 21 bases.

The CUL5-specific siRNA may have additional bases at 5'- or 3'-end. The length of the additional bases is generally about 2 to 4 bases. The additional bases may be DNA or RNA, but when DNA is used, the stability of the nucleic acid may be improved. Examples of the sequence of such additional bases include, but are not limited to, sequences such as ug-3', uu-3', tg-3', tt-3', ggg-3', guuu-3', gttt-3', ttttt-3', and uuuuu-3'.

The siRNA may have an overhang at the 3'-end, and specific examples thereof include those to which dTdT (dT represents deoxythymidine) is added. Alternatively, the siRNA may have a blunt end without terminal addition. In the siRNA, the number of bases in a sense strand may be different from that in an antisense strand, and examples of thereof include "asymmetrical interfering RNA (aiRNA)" in which the antisense strand has an overhang at the 3'-end and the 5'-end. Typical aiRNA is one in which the antisense strand consists of 21 bases, the sense strand consists of 15 bases, and an overhang structure of 3 bases is formed at each end of the antisense strand.

The position of the target sequence in the CUL5-specific siRNA is not particularly limited, but in one embodiment, it is desirable to select the target sequence from a region other than a 5'-UTR and from the initiation codon to about 50 bases, and a 3'-UTR. For the candidate group of the selected target sequence, it is preferable to examine whether there is no homology in a continuous 16-17 base sequence in mRNA other than the target using a homology search software such as BLAST (http://www.ncbi.nlm.nih.gov/BLAST/) to confirm the specificity of the selected target sequence. For the target sequence whose specificity has been confirmed, a double-stranded RNA consisting of a sense strand having a 3'-terminal overhang of TT or UU at 19-21 bases after AA (or NA) and an antisense strand having a sequence complementary to the 19-21 bases and a 3'-terminal overhang of TT or UU may be designed as the siRNA. In addition, the shRNA which is a precursor of the siRNA can be designed by appropriately selecting an arbitrary linker sequence (for example, about 5 to 25 bases) capable of forming a loop structure and linking the sense strand and the antisense strand via the linker sequence.

The sequence of the siRNA and/or the shRNA can be searched using various search software which is provided for free on web sites. Examples of such a site include the following. siRNA Target Finder (http://www.ambion.com/jp/techlib/misc/siRNA_finder.html), pSilencer (registered trademark) Insert design tool for Expression Vector (http://www.ambion.com/jp/techlib/misc/psilencer_converter.html) provided by Ambion, GeneSeer (http://codex.cshl.edu/scripts/newsearchhairpin.cgi) provided by RNAi Codex.

The siRNA can be prepared by synthesizing a sense strand and an antisense strand of the target sequence on mRNA with a DNA/RNA automatic synthesizer, respectively, denaturing the strands at about 90 to about 95°C for about 1 minute in an appropriate annealing buffer, and then annealing the resultant product at about 30 to about 70°C for about 1 to about 8 hours. Alternatively, the siRNA may also be prepared by synthesizing shRNA that is a precursor of the siRNA and cleaving the shRNA using an RNA cutting protein dicer.

The CUL5-specific miRNA is arbitrary as long as it inhibits translation of the CUL5 gene. For example, the miRNA may inhibit translation of target mRNA by pairing with a 3'-untranslated region (UTR) of the target, rather than cleaving the target mRNA like siRNA. The miRNA may be any of pri-miRNA (primary miRNA), pre-miRNA (precursor miRNA), and mature miRNA. The length of the miRNA is not particularly limited, and the length of the pri-miRNA is generally several hundred to several thousand bases, the length of the pre-miRNA is generally 50 to 80 bases, and the length of the mature miRNA is generally 18 to 30 bases. In one embodiment, the CUL5-specific miRNA is preferably pre-miRNA or mature miRNA, and more preferably mature miRNA. Such a CUL5-specific miRNA may be synthesized by a known procedure, or may be purchased from a company that provides the synthetic RNA.

The CUL5-specific antisense polynucleotide is a nucleic acid containing a base sequence complementary or substantially complementary to the base sequence of mRNA of the CUL5 gene or a part thereof, and has a function to form a specific and stable duplex with the mRNA and bind to the mRNA so as to suppress the synthesis of CUL5 protein. The antisense polynucleotide can be, for example, DNA, RNA, or a DNA/RNA chimera. In the case where the antisense polynucleotide is DNA, an RNA:DNA hybrid formed by a target RNA and the antisense DNA is recognized by endogenous ribonuclease H (RNase H) to cause selective degradation of the target RNA. Therefore, in the case of antisense DNA that directs the degradation with RNase H, the target sequence may be contained in mRNA as well as a sequence for an intron region in an initial translation product of the CUL5 gene. The intron sequence can be determined by comparing the genomic sequence with cDNA base sequence of the CUL5 gene using a homology search program such as BLAST or FASTA.

The length of the target region of the CUL5-specific antisense polynucleotide is not limited as long as the antisense polynucleotide hybridizes with the target region so as to inhibit translation into the CUL5 protein. The CUL5-specific antisense polynucleotide may be the entire sequence or a partial sequence of mRNA encoding CUL5. Oligonucleotides consisting of about 10 to about 40 bases, particularly about 15 to about 30 bases are preferable in consideration of ease of synthesis, and the problems of antigenicity and intracellular migration and the like, but are not limited thereto. More specifically, a 5'-terminal hairpin loop, a 5'-terminal untranslated region, a translation initiation codon, a protein coding region, an ORF translation stop codon, a 3'-terminal untranslated region, a 3'-terminal palindromic region or a 3'-terminal hairpin loop of the CUL5 gene may be selected as a preferred target region of the antisense polynucleotide, but the preferred target region is not limited thereto.

The CUL5-specific antisense polynucleotide may be one that can hybridize with mRNA and an initial transcript of the CUL5 gene to inhibit translation into protein, and can also bind to these genes that are double-stranded DNA to form a triplex to inhibit transcription into RNA (antigene).

The CUL5-specific siRNA, the CUL5-specific miRNA, the CUL5-specific antisense polynucleotide and the like can be prepared by determining mRNA or a target sequence of the initial transcript based on the cDNA sequence or the genomic DNA sequence of the CUL5 gene, and synthesizing a sequence complementary thereto using a commercially available DNA/RNA automatic synthesizer. Also, any antisense polynucleotide including various modifications can be chemically synthesized by a known method.

The expression cassette of a CUL5 gene expression suppressing polynucleotide (expression cassette 1) contains a promoter and a CUL5 gene expression suppressing polynucleotide coding sequence under the control of the promoter. The coding sequence is generally placed downstream of the promoter so as to be under the control of the promoter. Examples of the available promoter include RNA polymerase II (poII)-type promoters such as CMV promoters, EF1 promoters, SV40 promoters, MSCV promoters, hTERT promoters, β-actin promoters, and CAG promoters; RNA polymerase III (polIII)-type promoters such as mouse and human U6-snRNA promoters, human H1-RNase P RNA promoters, and human valine-tRNA promoters. Among these promoters, the polIII-type promoter is preferred from the viewpoint of the correct transcription ability of short RNA. The promoter is operably linked to the coding sequence. Here, the phrase "the promoter is operably linked to the coding sequence" has the same meaning as the phrase "the coding sequence is placed under the control of the promoter", and generally, the coding sequence is linked to the 3'-terminal side of the promoter directly or via another sequence. A polyA addition signal sequence is placed downstream of the coding sequence. Transcription is terminated by use of the polyA addition signal sequence. As the polyA addition signal sequence, a polyA addition sequence of SV40, a polyA addition sequence of a bovine-derived growth hormone gene, or the like can be used.

The polynucleotide of the present invention contains an expression cassette of a chimeric antigen receptor. CAR-expressing cells can be efficiently obtained by preparing CAR-expressing cells using the polynucleotide of the present invention containing the expression cassette of a chimeric antigen receptor.

The chimeric antigen receptor is not particularly limited as long as it can bind to the target antigen and can transmit a signal necessary for activation of the immune cell into the cell by binding of the target antigen. The chimeric antigen receptor typically includes an antigen-binding domain, a transmembrane domain, and an intracellular signal domain.

The antigen-binding domain is not particularly limited as long as it is a domain that is placed extracellularly when the chimeric antigen receptor is placed on the cell membrane and can recognize an antigen and bind to the antigen. Specific examples of the antigen include CD19, GD2, GD3, CD20, CD37, CEA, HER2, EGFR, type III mutant EGFR, CD38, BCMA, MUC-1, PSMA, WT1, cancer testis antigens (for example, NY-ESO-1, MAGE-A4, and the like), mutation peptides (for example, k-ras, h-ras, p53, and the like), hTERT, PRAM, TYRP1, mesothelin, PMEL, mucin, and further, complexes of these fragments with MHC (pMHC). Among them, in one embodiment of the present invention, the antigen is preferably CD19. The antigen-binding domain generally has one or more, preferably two, three, four, or five or more CDRs, or more preferably all of the six CDRs of an antibody or T cell receptor for an antigen (for example, in the case of CDRs of an antibody, a heavy chain CDR1, a heavy chain CDR2, a heavy chain CDR3, a light chain CDR1, a light chain CDR2, and a light chain CDR3). The antigen-binding region more preferably contains a variable region of an antibody or T cell receptor for an antigen (in the case of an antibody, for example, a heavy variable region and/or a light chain variable region).

The antigen-binding domain preferably has a single-chain antibody structure, and more preferably has an scFv structure. When the heavy chain variable region and the light chain variable region are included as in the scFv structure, the heavy chain variable region and the light chain variable region are generally linked via a linker. The linker is not particularly limited and is arbitrary as long as the antigen binding is not significantly impaired. The linker is preferably a linker composed of glycine or glycine and serine (GGS linker, GS linker, GGG linker, or the like). The length of the linker is not particularly limited. The number of amino acid residues of the linker is, for example, 5 to 30, and preferably 10 to 25. The arrangement relationship between the heavy chain variable region and the light chain variable region is not particularly limited, and either of an embodiment where the N-terminal side is the light chain variable region or a case where the N-terminal side is the heavy chain variable region can be adopted. The arrangement relationship is preferably the former.

The transmembrane domain is a domain placed in the cell membrane when the chimeric antigen receptor of the present invention is placed on the cell membrane, and is not particularly limited as long as it can constitute a chimeric antigen receptor. As the transmembrane domain, for example, a transmembrane region of CD28, CD3ε, CD8α, CD3, CD4, 4-1BB or the like can be used. The transmembrane region of each factor is known or can be easily determined from known sequence information (for example, using a prediction program of the transmembrane region or the like). In addition, a transmembrane domain including an artificially constructed polypeptide may be used. These transmembrane domains may be appropriately mutated as long as the function of the chimeric antigen receptor is not significantly inhibited.

As the transmembrane domain, a transmembrane region of CD28 can be preferably adopted. Specific examples of the transmembrane region of CD28 include the amino acid sequence described in (a) below or (b) below:
(a) an amino acid sequence set forth in SEQ ID NO: 3, or
(b) an amino acid sequence having equal to or greater than 85% identity to the amino acid sequence set forth in SEQ ID NO: 3 and capable of being placed in the cell membrane when the chimeric antigen receptor of the present invention is placed on the cell membrane.

In (b) above, the identity is preferably 90% or more, more preferably 95% or more, still more preferably 98% or more, and particularly preferably 99% or more.

An example of the amino acid sequence described in (b) above is, for example,
(b') an amino acid sequence having a substitution, deletion, addition, or insertion of one or more amino acids with respect to the amino acid sequence set forth in SEQ ID NO: 3 and capable of being placed in the cell membrane when the chimeric antigen receptor of the present invention is placed on the cell membrane.

In (b') above, "a plurality of" means, for example, 2 to 5, preferably 2 to 3, and more preferably 2.

In one embodiment of the present invention, the antigen-binding domain and the transmembrane domain are preferably linked directly or via a relatively short spacer. In the chimeric antigen receptor of the present invention, the number of constituent amino acid residues between the antigen-binding domain and the transmembrane domain can be, for example, 400 or less, 300 or less, or 250 or less, and is particularly preferably 100 or less. The number of constituent amino acid residues is preferably 0 to 60, more preferably 0 to 40, and still more preferably 0 to 30. In a particularly preferred embodiment of the present invention, the number of amino acid residues is 1 or more, 4 or more, or 8 or more, and 25 or less, 20 or less, or 15 or less, and 1 to 25, 4 to 20, or 8 to 15. The sequence of the spacer is not particularly limited, and for example, a sequence such as a hinge portion of IgG, preferably human IgG (for example, subtype IgG1 or IgG4) or a part thereof, a hinge portion and a part of CH2, or a part of a factor used for a transmembrane domain (for example, CD28) can be used as the spacer. Incidentally, it can be expected that a spacer having high flexibility is configured by using the sequence of the hinge portion or a part thereof.

The intracellular signal domain is a domain that is placed in a cell when the chimeric antigen receptor of the present invention is placed on the cell membrane, and is capable of transmitting a signal necessary for exerting an effector function of an immune cell, that is, transmitting a signal necessary for activation of an immune cell when the antigen-binding domain binds to an antigen. The intracellular signal domain preferably includes an intracellular activation domain. As the intracellular activation domain, for example, an intracellular domain, such as FcεRIγ in addition to CD3ζ, can be used. Preferably, CD3ζ is used. CD3 may be appropriately mutated as long as the function of the antigen receptor is not inhibited. Mutation of CD3 is preferably made such that CD3 contains ITAM (immunoreceptor tyrosine-based activation motif).

Specific examples of the intracellular activation domain include an amino acid sequence described in (c) below or an amino acid sequence described in (d) below:
(c) an amino acid sequence set forth in SEQ ID NO: 5, or
(d) an amino acid sequence having equal to or greater than 85% identity to the amino acid sequence set forth in SEQ ID NO: 5 and having an immune cell activation effect.

In (d) above, the identity is preferably 90% or more, more preferably 95% or more, still more preferably 98% or more, and particularly preferably 99% or more.

An example of the amino acid sequence described in (d) above is, for example,
(d') an amino acid sequence having a substitution, deletion, addition, or insertion of one or more amino acids with respect to the amino acid sequence set forth in SEQ ID NO: 5 and having an immune cell activation action.

In (d') above, "a plurality of" means, for example, 2 to 15, preferably 2 to 10, more preferably 2 to 5, and still more preferably 2 to 3.

The intracellular signal domain preferably includes an intracellular domain of a co-stimulator. The intracellular domain of a co-stimulator is not particularly limited as long as it is an intracellular domain derived from a co-stimulator of a T cell or the like. For example, one or more intracellular domains selected from the group consisting of OX40, 4-1BB, GITR, CD79a, CD40, CD27, CD278, CD28, and the like can be appropriately selected and used. In one embodiment of the present invention, a domain in which a CD79a intracellular domain and a CD40 intracellular domain are linked in tandem can be adopted.

Specific examples of the intracellular domain include an amino acid sequence described in (e) below or an amino acid sequence described in (f) below:
(e) an amino acid sequence set forth in any of SEQ ID NOs: 4 and 30 to 32; or
(f) an amino acid sequence having equal to or greater than 85% identity to the amino acid sequence set forth in any of SEQ ID NOs: 4 and 30 to 32 and having an immune cell activation effect.

In (f) above, the identity is preferably 90% or more, more preferably 95% or more, still more preferably 98% or more, and particularly preferably 99% or more.

An example of the amino acid sequence described in (f) above is, for example,
(f') an amino acid sequence having a substitution, deletion, addition, or insertion of one or more amino acids with respect to the amino acid sequence set forth in SEQ ID NO: 4 and having an immune cell activation action.

In (f') above, "a plurality of" means, for example, 2 to 15, preferably 2 to 10, more preferably 2 to 5, and still more preferably 2 to 3.

The chimeric antigen receptor of the present invention can include other regions other than those described above. Examples of the other regions include a leader sequence (signal peptide) to facilitate transport of the CAR onto the cell membrane (for example, the leader sequence of a GM-CSF receptor), and spacer/linker (for example, between the transmembrane region and the intracellular signal domain, between each domain in the intracellular signal domain).

There have been several reports of experiments, clinical studies, and the like using CARs (for example, Rossig C, et al. Mol Ther 10: 5-18, 2004; Dotti G, et al. Hum Gene Ther 20: 1229-1239, 2009; Ngo MC, et al. Hum Mol Genet 20 (R1): R93-99, 2011; Ahmed N, et al. Mol Ther 17: 1779-1787, 2009; Pule MA, et al. Nat Med 14: 1264-1270, 2008; Louis CU, et al. Blood118: 6050-6056, 2011; Kochenderfer JN, et al. Blood 116: 4099-4102, 2010; Kochenderfer JN, et al. Blood 119: 2709-2720, 2012; Porter DL, et al. N Engl J Med 365: 725-733, 2011; Kalos M, et al. Sci Transl Med 3: 95ra73, 2011; Brentjens RJ, et al. Blood 118: 4817-4828, 2011; Brentjens RJ, et al. Sci Transl Med 5: 177ra38, 2013). The chimeric antigen receptor of the present invention can be constructed with reference to these reports.

A particularly preferred embodiment of the chimeric antigen receptor of the present invention includes a chimeric antigen receptor including a core region in which an antigen-binding domain, a transmembrane domain, and an intracellular signal domain are arranged in this order. Each domain is linked directly or via a spacer/linker.

The chimeric antigen receptor of the present invention may have a protein or peptide, such as a known protein tag or signal sequence, added. Examples of the protein tag include biotin, His tags, FLAG tags, Halo tags, MBP tags, HA tags, Myc tags, V5 tags, PA tags, and fluorescent protein tags.

The expression cassette of the chimeric antigen receptor (expression cassette 2) contains a promoter and a chimeric antigen receptor coding sequence under the control of the promoter. The coding sequence is generally placed downstream of the promoter so as to be under the control of the promoter. Examples of the available promoter include RNA polymerase II (poII)-type promoters such as CMV promoters, EF1 promoters, SV40 promoters, MSCV promoters, hTERT promoters, β-actin promoters, and CAG promoters. The promoter is operably linked to the coding sequence. Here, the phrase "the promoter is operably linked to the coding sequence" has the same meaning as the phrase "the coding sequence is placed under the control of the promoter", and generally, the coding sequence is linked to the 3'-terminal side of the promoter directly or via another sequence. A polyA addition signal sequence is placed downstream of the coding sequence. Transcription is terminated by use of the polyA addition signal sequence. As the polyA addition signal sequence, a polyA addition sequence of SV40, a polyA addition sequence of a bovine-derived growth hormone gene, or the like can be used.

The polynucleotide of the present invention may contain other sequences in addition to the expression cassette 1 and the expression cassette 2. Other sequences include enhancer sequences, repressor sequences, insulator sequences, origins of replication, reporter protein (for example, fluorescent protein and the like) coding sequences, drug-resistant-gene-coding sequences, and the like.

The expression cassette may contain a detection gene (a reporter gene, a cell-specific or tissue-specific gene, a selection marker gene, or the like), an enhancer sequence, a WRPE sequence, or the like. The gene for detection is used for determination of success or failure or efficiency of introduction of the expression cassette, detection of CAR gene expression or determination of expression efficiency, or selection and sorting of cells in which the CAR gene is expressed. On the other hand, the use of an enhancer sequence improves the expression efficiency. As the gene for detection, it is possible to use genes such as neo gene that confers resistance to neomycin, an npt gene (Herrera Estrella, EMBO J. 2 (1983), 987-995) and an nptII gene (Messing & Vierra. Gene 1 9: 259-268 (1982)) that confer resistance to kanamycin and the like, a hph gene that confers resistance to hygromycin (Blochinger & Diggl mann, Mol Cell Bio 4: 2929-2931), a dhfr gene that confers resistance to methotrexate (Bourouis et al., EMBO J. 2(7)), and the like (the above are marker genes), genes of fluorescent proteins such as a luciferase gene (Giacomin, P1. Sci. 116 (1996), 59 to 72; Scikantha, J. Bact. 178 (1996), 121), a β-glucuronidase (GUS) gene, GFP (Gerdes, FEBS Lett. 389 (1996), 44-47) and their variants (EGFP, d2EGFP, and the like) (the above are reporter genes), and an epidermal growth factor receptor (EGFR) gene lacking an intracellular domain. The gene for detection is linked to the CAR gene, for example, via a bicistronic control sequence (for example, internal ribosome entry site (IRES)) or a sequence encoding a self-cleaving peptide. Examples of the self-cleaving peptide are 2A peptides (T2A) derived from Thosea asigna virus, but are not limited thereto. As the self-cleaving peptide, 2A peptide derived from foot-and-mouth disease virus (FMDV) (F2A), 2A peptide derived from equine rhinitis A virus (ERAV) (E2A), 2A peptide derived from porcine teschovirus (PTV-1) (P2A), and the like are known.

The polynucleotide of the present invention may be a linear polynucleotide or a cyclic polynucleotide (such as a vector). The vector may be a plasmid vector or a viral vector. The vector may be, for example, a cloning vector or an expression vector. Examples of the expression vector include vectors for prokaryotic cells such as E. coli or actinomycetes, and vectors for eukaryotic cells such as yeast cells, insect cells, or mammalian cells. More specifically, examples of the viral vector include retroviral vectors, lentiviral vectors, adenoviral vectors, adeno-associated viral vectors, herpesviral vectors, and Sendai viral vectors, and examples of non-viral vector include various plasmid vectors, liposomal vectors, positively charged liposomal vectors (Felgner, P.L., Gadek, T.R., Holm, M. et al., Proc. Natl. Acad. Sci., 84: 7413-7417, 1987), YAC vectors, and BAC vectors.

The polynucleotide of the present invention is preferably a viral vector or a viral genome from the viewpoint of being able to reduce the damage given to the cell when obtaining the cell of the present invention described later. The polynucleotide of the present invention is more preferably a retroviral vector, or a retroviral genome, or a lentiviral vector, or a lentiviral genome. The polynucleotide of the present invention is particularly preferably a lentiviral vector or a lentiviral genome.

### 3. Cell

In one embodiment, the present invention relates to a cell containing the polynucleotide of the present invention (sometimes referred to as "cell 1 of the present invention" in the present specification). Also, in one embodiment, the present invention relates to a cell which has been modified so as to reduce expression of CUL5 gene and expresses a chimeric antigen receptor (sometimes referred to as "cell 2 of the present invention" in the present specification). In the present specification, the cell 1 of the present invention and the cell 2 of the present invention are sometimes collectively referred to as "the cell of the present invention". These cells will be described below.

### 3-1. Cell 1 of present invention

The cell 1 of the present invention can be obtained by a method including introducing the polynucleotide of the present invention into a cell. In one embodiment of the cell, the expression of CUL5 gene is reduced by a CUL5 gene expression suppressing polynucleotide, and the cell expresses a chimeric antigen receptor. The expression level of Cullin-5 protein in the cell 1 of the present invention can be, for example, 70% or less, 60% or less, 50% or less, 40% or less, 30% or less, 20% or less, or 10% or less with respect to the expression level of Cullin-5 protein in a control cell (a cell treated under the same conditions except that the polynucleotide of the present invention is not introduced).

The method for introducing the polynucleotide of the present invention into a cell is not particularly limited, but is preferably a method of introducing a virus containing the polynucleotide of the present invention from the viewpoint of reducing damage to the cell and further enhancing the preparation efficiency and proliferation capability of CAR-expressing cells. As a result, the polynucleotide of the present invention can be introduced without using a method that may damage the cell, such as an electroporation method or a lipofection method.

Examples of the cells (cells for CAR expression) into which the polynucleotide of the present invention is introduced include CD4-positive CD8-negative T cells, CD4-negative CD8-positive T cells, T cells prepared from iPS cells, αβ-T cells, γδ-T cells, NK cells, and NKT cells. Various cell populations can be used as long as they contain lymphocytes or progenitor cells as described above. PBMCs (peripheral blood mononuclear cells) collected from peripheral blood are one of preferred target cells. That is, in a preferred embodiment, a gene transfer operation is performed on PBMCs. PBMCs can be prepared according to or in accordance with an ordinary method.

It is preferable to activate the cells for CAR expression before the introduction of the polynucleotide of the present invention. For example, the cells for CAR expression can be activated by stimulation with an anti-CD3 antibody and an anti-CD28 antibody. For example, the stimulation with an anti-CD3 antibody and an anti-CD28 antibody can be applied by culturing in a culture container (for example, a culture dish) having a culture surface coated with an anti-CD3 antibody and an anti-CD28 antibody. The stimulation can also be performed using magnetic beads coated with an anti-CD3 antibody and an anti-CD28 antibody (for example, Dynabeads T-Activator CD3/CD28 provided by Veritas Corporation).

In order to increase the survival rate/proliferation rate of cells, it is preferable to use a culture solution to which a T-cell growth factor is added in the activation treatment. As the T-cell growth factor, IL-2, IL-15, IL-7, and the like can be used. T-cell growth factors such as IL-2, IL-15, and IL-7 can be prepared according to a conventional method. Commercially available products can also be used. The use of T-cell growth factors of animals other than humans is not excluded, but generally, T-cell growth factors derived from humans (which may be recombinant) are used.

Typically, for their application (administration to a patient), cells after introduction of the polynucleotide of the present invention (polynucleotide-introduced lymphocytes of the present invention) are propagated. For example, the polynucleotide-introduced lymphocytes of the present invention are cultured (subcultured as necessary) using a culture solution to which a T-cell growth factor has been added. In addition to this culture, the same treatment (reactivation) as in the case of activation of the cells for CAR expression may be performed.

Although a medium to which serum (human serum, fetal bovine serum, or the like) is added may be used for culturing the cells for CAR expression and the polynucleotide-introduced lymphocytes of the present invention, by using a serum-free medium, it is possible to prepare cells having advantages of high safety in clinical application and occurrence of a difference in culture efficiency due to a difference between serum lots being unlikely. When serum is used, it is preferable to use autologous serum, i.e., serum collected from a patient to whom CAR-transgenic lymphocytes are administered.

### 3-2. Cell 2 of present invention

The cell 2 of the present invention can be obtained by modifying the cell to reduce expression of CUL5 gene, and further expressing a chimeric antigen receptor. The expression level of Cullin-5 protein in the cell 2 of the present invention can be, for example, 70% or less, 60% or less, 50% or less, 40% or less, 30% or less, 20% or less, or 10% or less with respect to the expression level of Cullin-5 protein in a control cell (a cell treated under the same conditions except that the cell is not modified to reduce expression of CUL5 gene). By reducing the expression of CUL5 gene, the proliferation capability of CAR-expressing cells can be enhanced. Examples of the modification to reduce the expression of CUL5 gene include a gene defect (gene disruption), a mutation in a protein coding region, a mutation in a splicing regulatory region, and a mutation in an expression control region (for example, a promoter, an activator, an enhancer, or the like). The modification can be performed using a gene editing system such as a CRSPR/Cas system.

The method for expressing a chimeric antigen receptor is not particularly limited. For example, a chimeric antigen receptor can be expressed by introducing a polynucleotide containing an expression cassette of the chimeric antigen receptor into a cell.

For matters not described above, the description of "3-1. Cell 1 of present invention" is cited.

### 3-3. Cell of present invention

Cells from which the cell of the present invention is derived are not particularly limited. For the purpose of using the cell of the present invention in purification of the chimeric antigen receptor of the present invention, examples of the derived cell include cells that can be used for protein expression (for example, insect cells, eukaryotic cells, mammalian cells, and the like).

The cell of the present invention is preferably a lymphocyte cell (for example, a T cell (for example, a CD4-positive CD8 negative T cell, a CD4-negative CD8-positive T cell, a T cell prepared from iPS cells, an αβ-T cell, a γδ-T cell, or the like), an NK cell, an NKT cell, or the like). These cells are preferably cells expressing the chimeric antigen receptor of the present invention, and in a more specific embodiment, these cells express the chimeric antigen receptor of the present invention on the cell membrane, and preferably express the chimeric antigen receptor of the present invention in a state where the antigen-binding domain is exposed outside the cell membrane.

A T cell or the like expressing the chimeric antigen receptor recognizes an antigen in an antigen-binding region, and then transmits a recognition signal to the inside of the T cell or the like to activate a signal for inducing cytotoxic activity, and in conjunction with this, the T cell or the like can mount attacks against other cells or tissues in which the cell expresses the antigen or exhibit cytotoxic activity.

When the cell exhibiting such a function is a CTL, the cell is called a chimeric antigen receptor T cell (CAR-T cell). Cells that have potential to exhibit cytotoxic activity, such as NK cells, can also exhibit cytotoxic activity when the antigen-binding region binds to the antigen, as with the chimeric antigen receptor T cell. Thus, a host cell containing the polynucleotide encoding the chimeric antigen receptor of the present invention (in particular, a host cell having cytotoxic activity) is useful as an active ingredient of pharmaceutical compositions.

### 4. Pharmaceutical composition

In one embodiment, the present invention relates to a pharmaceutical composition containing the cell of the present invention (sometimes referred to as "the pharmaceutical composition of the present invention" in the present specification). The pharmaceutical composition of the present invention will be described below.

The pharmaceutical composition of the present invention can be widely used as a cell preparation for treatment, prevention, or improvement of a tumor/cancer (target disease) expressing a target antigen of an antigen-binding domain. Target diseases include solid cancers and hematological cancers. Examples of the target disease include various B-cell malignant lymphomas (B-cell acute lymphocytic leukemia, follicular lymphoma, diffuse lymphoma, mantle cell lymphoma, MALT lymphoma, intravascular B-cell lymphoma, CD20-positive Hodgkin lymphoma, and the like), myeloproliferative diseases, myelodysplastic/myeloproliferative tumors (CMML, JMML, CML, MDS/MPN-UC), myelodysplastic syndrome, acute myeloid leukemia, multiple myeloma, lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, and rhabdomyosarcoma. "Treatment" includes alleviation (moderation) of symptoms or associated symptoms characteristic to the target disease, inhibition or delay of deterioration of symptoms, and the like. "Prevention" refers to prevention or delay of the development/expression of a disease (disorder) or symptoms thereof, or reduction of the risk of development/expression. On the other hand, "improvement" refers to alleviation (moderation), change for the better, remission, or cure (including partial cure) of a disease (disorder) or symptoms thereof.

The pharmaceutical composition of the present invention contains a therapeutically effective amount of the cells of the present invention. For example, 1 × 10⁴ to 1 × 10¹⁰ cells can be contained for one administration. The following components can be contained in the cell preparation: dimethylsulfoxide (DMSO) or serum albumin for the purpose of cell protection; antibiotics for the purpose of preventing bacterial contamination; and various components (vitamins, cytokine, growth factors, steroids, and the like) for the purpose of activation, proliferation, or differentiation induction of cells.

The administration route of the CAR-transgenic lymphocytes or cell preparation of the present invention is not particularly limited. For example, it is administered by intravenous injection, intraarterial injection, portal vein injection, intradermal injection, subcutaneous injection, intramuscular injection, or intraperitoneal injection. Local administration may be used in place of systemic administration. Examples of the local administration include direct injection into target tissues, body parts, or organs. The administration schedule may be made according to the sex, age, body weight, and pathology of the subject (patient), and the like. In addition to single administration, multiple administrations may be performed continuously or periodically.

### Examples

The present invention will be described in detail below with reference to Examples. However, the present invention is not limited to these Examples.

### Test Example 1. Design and preparation of CAR construct

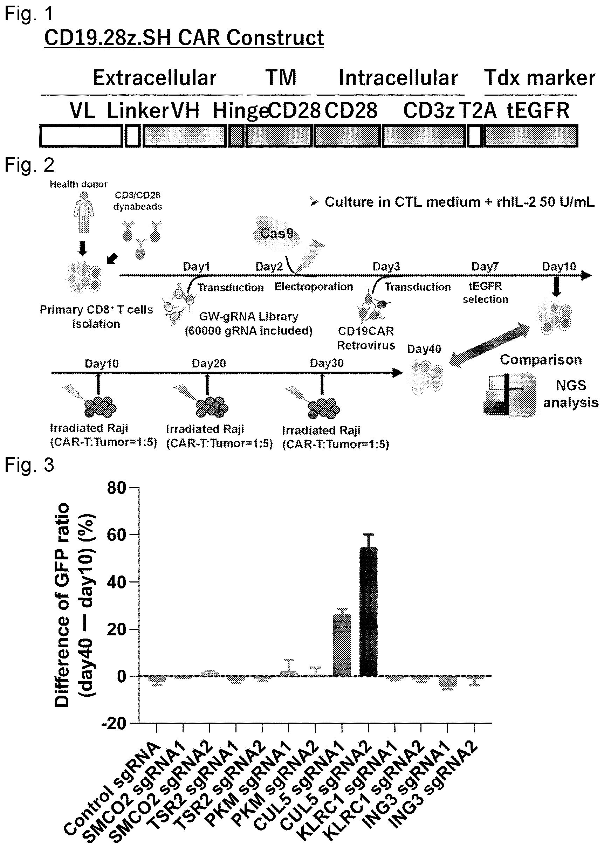

A CAR construct (Fig. 1; amino acid sequence: SEQ ID NO: 8; base sequence: SEQ ID NO: 16) containing
an anti-CD19 scFv domain (amino acid sequence: SEQ ID NO: 1, base sequence: SEQ ID NO: 9),
an IgG4 hinge region (amino acid sequence: SEQ ID NO: 2, base sequence: SEQ ID NO: 10),
a CD28 transmembrane domain (amino acid sequence: SEQ ID NO: 3, base sequence: SEQ ID NO: 11),
a CD28 intracellular domain (amino acid sequence: SEQ ID NO: 4, base sequence: SEQ ID NO: 12),
a CD3ζ intracellular domain (amino acid sequence: SEQ ID NO: 5, base sequence: SEQ ID NO: 13),
a 5 amino acid spacer,
a T2A sequence (amino acid sequence: SEQ ID NO: 6, base sequence: SEQ ID NO: 14), and
a truncated EGFR domain (amino acid sequence: SEQ ID NO: 7; base sequence: SEQ ID NO: 15) arranged in this order from the N-terminal side was designed, and the coding sequence of the CAR (base sequence: SEQ ID NO: 16) was incorporated into a retroviral vector (vector name: pLZRS-BMN-Z) using restriction enzyme recognition sequences added to both ends thereof.

The sequences of the anti-CD19 scFv domain and the truncated EGFR domain contain a GM-CSF receptor leader sequence added to the N-terminus.

### Test Example 2. Screening of CAR-T-cell growth factors

Screening of CAR-T-cell growth factors was performed by GW (Genome Wide)-CRISPR screening. The outline is shown in Fig. 2. Specifically, the screening was performed as follows.

Blood was collected from three healthy donors, CD8-positive lymphocytes were sorted using immunomagnetic beads (Myltenii), and the CD8-positive cells were stimulated with CD3/CD28 beads to start proliferation (Day0). A lentivirus expressing GW-gRNA was transfected on Day1, and Cas9 protein was introduced by electroporation on Day2. On Day3, a retrovirus in which a CAR construct containing a CD19CAR and EGFR (truncated EGFR, tEGFR) lacking an intracellular domain as a transfection marker (Test Example 1) was packaged was transfected. On Day7, using tEGFR as a transfection marker, tEGFR-positive cells (CD19CAR-positive cells) were purified using a biotinylated EGFR antibody and anti-biotin microbeads. The culture continued until Day10, and thereafter, repeated stimulation was performed 3 times using Raji cell line (CD19+, Burkitt's lymphoma-derived cell line) irradiated with 100 Gy every 10 days, and cells remaining on Day40 were collected. The gRNA sequence was determined and quantified using DNA collected from the CAR-T cells on Day10 and the CAR-T cells on Day40. Ranking of knock-out genes per donor was performed using GFOLD analysis. GFOLD analysis was integrated, and six genes were extracted as genes reported to be expressed in T cells within the top 10.

Subsequently, secondary screening was performed. CAR-T cells were prepared according to the scheme of Fig. 2, except that a lentiviral vector containing an expression cassette of either of the two gRNAs targeting each of the six genes extracted above (vector name: pLV-EGFP-U6) was used in place of the GW-gRNA Library. The target sequences of gRNA are as follows.
SMCO2 gene (NCBI gene ID: 341346)
   gRNA1: SEQ ID NO: 17
   gRNA2: SEQ ID NO: 18
TSR2 gene (NCBI gene ID: 90121)
   gRNA1: SEQ ID NO: 19
   gRNA2: SEQ ID NO: 20
PKM gene (NCBI gene ID: 5315)
   gRNA1: SEQ ID NO: 21
   gRNA2: SEQ ID NO: 22
CUL5 gene (NCBI gene ID: 8065)
   gRNA1 (#4690): SEQ ID NO: 23
   gRNA2 (#8377): SEQ ID NO: 24
KLRC1 gene (NCBI gene ID: 3821)
   gRNA1: SEQ ID NO: 25
   gRNA2: SEQ ID NO: 26
ING3 gene (NCBI gene ID: 54556)
   gRNA1: SEQ ID NO: 27
   gRNA2: SEQ ID NO: 28

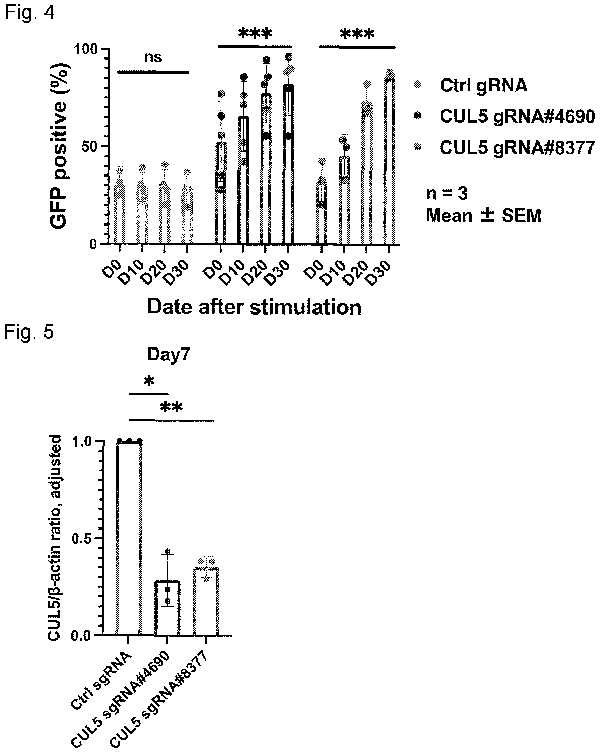

The results are shown in Figs. 3 and 4. Only when CUL5 was suppressed, the GFP-positive rate increased. The results of measuring the expression level of CUL5 on Day7 by Western blotting are shown in Fig. 5.

### Test Example 3. Analysis of effect of CUL5 knockout 1

CAR-T cells were prepared according to the scheme of Fig. 2, except that cells from each of five donors was used and a lentiviral vector containing an expression cassette of either of the two gRNAs targeting CUL5 (Test Example 2) was used in place of the GW-gRNA Library.

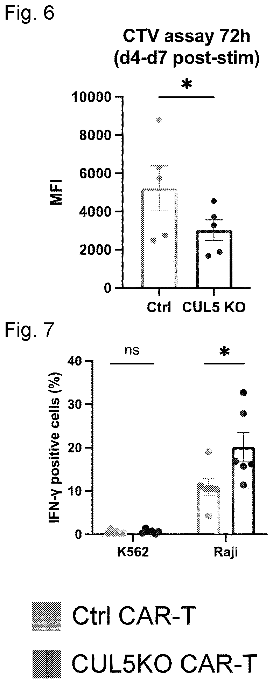

After stimulation with irradiated Raji cells on Day10, CAR-T cells on Day 4 (Day 14 in total) were stained with CellTrace Violet, and the degree of cell division after 72 hours (Day 17 in total) was measured by dilution of CellTrace Violet using flow cytometry. The results are shown in Fig. 6. It was found that cell division was enhanced by CUL5 knockout.

Intracellular cytokine production after stimulation was measured by flow cytometry with intracellular cytokine staining. Control sgRNA-introduced CAR-T and CUL5KO CAR-T were stimulated with K562 (CD19 negative) and Raji (CD19 positive), respectively, and intracellular cytokine (IFN-γ) after 4 hours was stained. The results are shown in Fig. 7. It was found that cytokine production was enhanced by CUL5 knockout.

### Test Example 4. Analysis of effect of CUL5 knockout 2

CAR-T cells were prepared according to the scheme of Fig. 2, except that cells from each of four donors was used and a lentiviral vector containing an expression cassette of either of the two gRNAs targeting CUL5 (Test Example 2) was used in place of the GW-gRNA Library.

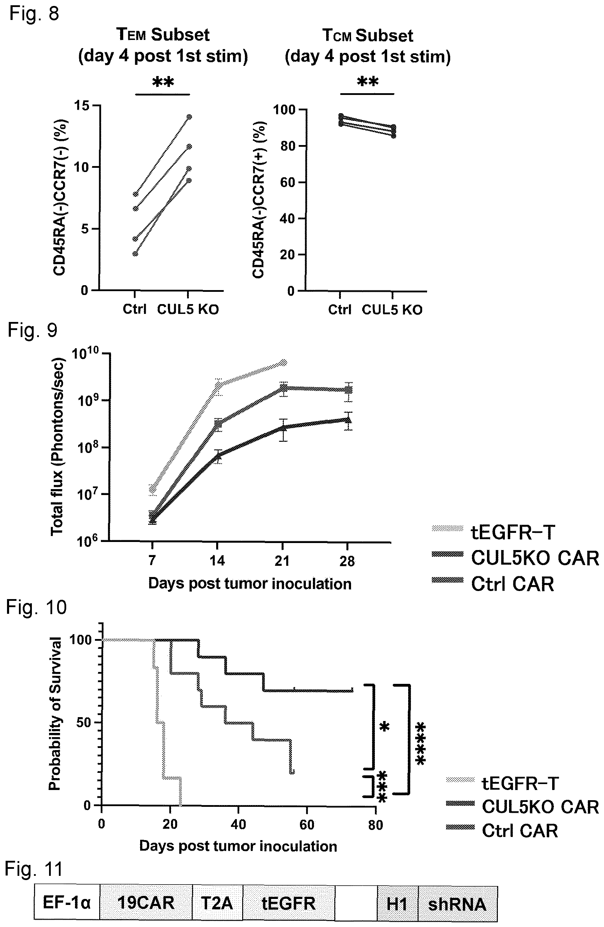

After initial stimulation of CAR-T cells on Day10 with CD3/28 beads and Raji cells, surface characteristics on Day 4 were measured by flow cytometry, and the proportions of effector memory T cells (T_{EM}: CD45RA(-)CCR7(-)) and central memory T cells (T_{CM}: CD45RA(-)CCR7(+)) were calculated. The results are shown in Fig. 8. It was found that the number of effector memory T cells was increased by CUL5 knockout.

### Test Example 5. Analysis of effect of CUL5 knockout 3

CAR-T cells were prepared according to the scheme of Fig. 2, except that a lentiviral vector containing an expression cassette of either of the two gRNAs targeting CUL5 (Test Example 2) was used in place of the GW-gRNA Library.

NOD-Scid shi common gamma chain knockout mice (NOG mice) were intravenously injected with Raji cells constitutively expressing firefly luciferase and GFP (Raji/Luc cells) at 5.0x10e5 on Day0, and various T cells (CAR-T cells expressing control gRNA, CAR-T cells expressing CUL5 gRNA, and T cells expressing tEGFR) were intravenously administered at 1.0x10e6 on Day7. Thereafter, luciferin was administered every week, and luminescence of tumor cells was photographed.

The results are shown in Figs. 9 and 10. In the CAR-T cells, bioluminescent showed a low value over time and survival was also significantly good by CUL5 knockout. Experiments were performed using tEGFR-T, n = 6; control CAR-T and CUL5KO CAR-T, n = 10, and T cells generated from three different donors.

### Test Example 6. Design and preparation of Two-in-one vector 1

A CAR expression cassette containing
an anti-CD19 scFv domain (amino acid sequence: SEQ ID NO: 1, base sequence: SEQ ID NO: 9),
an IgG4 hinge region (amino acid sequence: SEQ ID NO: 2, base sequence: SEQ ID NO: 10),
a CD28 transmembrane domain (amino acid sequence: SEQ ID NO: 3, base sequence: SEQ ID NO: 11),
a CD28 intracellular domain (amino acid sequence: SEQ ID NO: 4, base sequence: SEQ ID NO: 12),
a CD3ζ intracellular domain (amino acid sequence: SEQ ID NO: 5, base sequence: SEQ ID NO: 13),
a 5 amino acid spacer,
a T2A sequence (amino acid sequence: SEQ ID NO: 6, base sequence: SEQ ID NO: 14), and
a truncated EGFR domain (amino acid sequence: SEQ ID NO: 7; base sequence: SEQ ID NO: 15) arranged in this order from the N-terminal side, and
an expression cassette of shRNA (base sequence: SEQ ID NO: 29) against CUL5
were each incorporated into a lentiviral vector (vector name: pGreenPuro_shRNA(EF1)) using restriction enzyme recognition sequences added to both ends thereof. A promoter of the CAR expression cassette is an EF-1α promoter and a promoter of the shRNA expression cassette is an H1 promoter. A schematic diagram of the resulting CAR construct is shown in Fig. 11. The sequences of the anti-CD19 scFv domain and the truncated EGFR domain contain a GM-CSF receptor leader sequence added to the N-terminus.

### Test Example 7. Analysis of effect of Two-in-one vector

### Method 1

CAR-T cells were prepared according to the scheme of Fig. 2, except that cells from each of three donors was used and a lentiviral vector containing an expression cassette of either of the two gRNAs targeting CUL5 (Test Example 2) was used in place of the GW-gRNA Library.

### Method 2

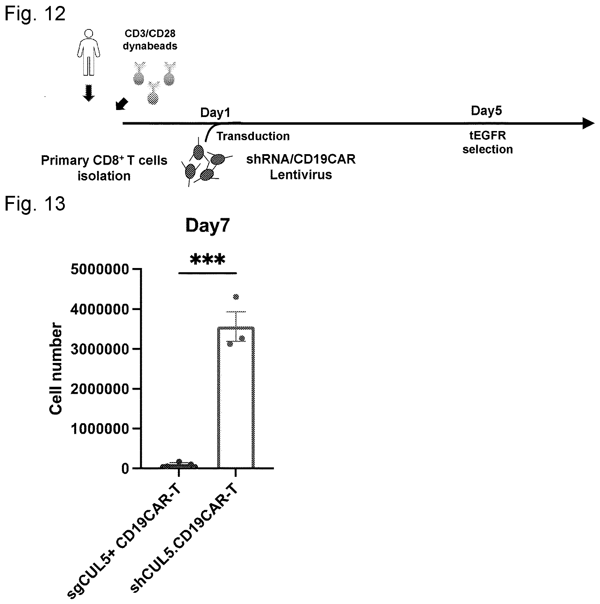

On the other hand, a part of the scheme in Fig. 2 (scheme before stimulation start) was changed to the scheme in Fig. 12 to prepare CAR-T cells. Specifically, blood was collected from three healthy donors, CD8-positive lymphocytes were sorted using immunomagnetic beads (Myltenii), and the CD8-positive cells were stimulated with CD3/CD28 beads to start proliferation (Day0). A lentivirus obtained from two-in-one vector (Test Example 6) was transfected on Day1. On Day5, using tEGFR as a transfection marker, tEGFR-positive cells (CD19CAR-positive cells) were purified using a biotinylated EGFR antibody and anti-biotin microbeads. The culture continued until Day10, and thereafter, repeated stimulation was performed 3 times using Raji cell line (CD19+, Burkitt's lymphoma-derived cell line) irradiated with 100 Gy every 10 days, and cells remaining on Day40 were collected.

### Method 3

In addition, as a control of Method 2, CAR-T cells were prepared in the same manner as in Method 2, except that a vector containing an expression cassette of shRNA against GFP was used in place of the expression cassette of shRNA against CUL5.

For any of the above methods, preparation of CAR-T cells was started from 1x10e6 CD8-positive cells.

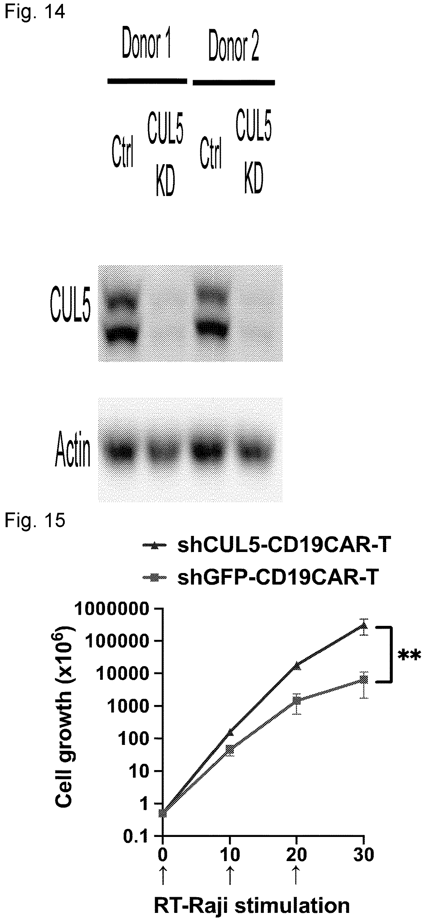

The number of CAR-T cells was measured by trypan blue staining. The number of CAR-T cells on Day7 is shown in Fig. 13. The results of measuring the expression level of CUL5 on Day10 by Western blotting are shown in Fig. 14. The proliferation of CAR-T cells is also shown in Fig. 15. It has been found that the preparation efficiency and proliferation of CAR-T cells can be greatly improved by preparing CUL5 knockdown CAR-T cells using a polynucleotide containing an expression cassette of a CUL5 gene expression suppressing polynucleotide and an expression cassette of a chimeric antigen receptor as in Method 2.

### Test Example 8. Analysis of effect of Two-in-one vector 2

CAR-T cells were prepared according to Method 2 and Method 3 of Test Example 7.

NOD-Scid shi common gamma chain knockout mice (NOG mice, n = 8 to 10) were intravenously injected with Raji cells constitutively expressing firefly luciferase and GFP (Raji/Luc cells) at 2.0x10e6 on Day0, and various T cells were intravenously administered at 1.0x10e6 on Day7. Thereafter, luciferin was administered every week, and luminescence of tumor cells was photographed.

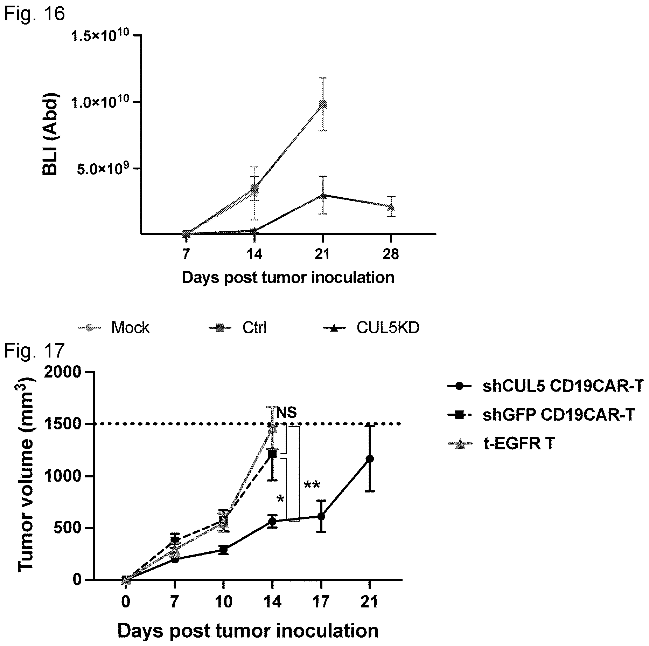

The results are shown in Fig. 16. In the CAR-T cells knocked down for CUL5 by two-in-one vector, bioluminescent showed a low value over time.

### Test Example 9. Analysis of effect of Two-in-one vector 3

CAR-T cells were prepared according to Method 2 and Method 3 of Test Example 7.

NOD-Scid shi common gamma chain knockout mice (NOG mice) were intravenously injected with Raji cells constitutively expressing firefly luciferase and GFP (Raji/Luc cells) at 2.0x10e6 on Day0, and various T cells were intravenously administered at 1.0x10e6 on Day10. Thereafter, luciferin was administered every week, luminescence of tumor cells was photographed, and the tumor volume was measured. When the tumor volume exceeded 1500mm3, the mouse was subjected to euthanasia treatment.

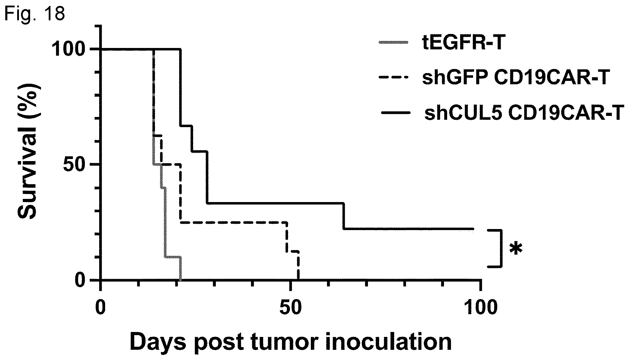

The measurement results of the tumor volume are shown in Fig. 17, and the survival rate is shown in Fig. 18. In the CAR-T cells knocked down for CUL5 by two-in-one vector, the tumor growth was remarkably suppressed, and also the survival rate was greatly improved.

### Test Example 10. Design and preparation of Two-in-one vector 2

A lentiviral vector was obtained in the same manner as in Test Example 6, except that an anti-Eval scFv domain was adopted in place of the anti-CD19 scFv domain, and a tandem-linked domain of a 4-1BB intracellular domain (amino acid sequence: SEQ ID NO: 30, base sequence: SEQ ID NO: 33) or a CD79a intracellular domain (amino acid sequence: SEQ ID NO: 31, base sequence: SEQ ID NO: 34)-CD40 intracellular domain (amino acid sequence: SEQ ID NO: 32, base sequence: SEQ ID NO: 35) was adopted in place of the CD28 intracellular domain as the intracellular domain.

### Test Example 11. Analysis of effect of Two-in-one vector 4

CAR-T cells were prepared in the same manner as in Method 2 and Method 3 of Test Example 7 except for using the two-in-one vector of Test Example 10.

NCI-H1975 (NCI-H1975-firefly luciferase-GFP, cells constitutively expressing firefly luciferase and GFP) was injected subcutaneously in mice at 1x10e6. After 14 days, various T cells (shCUL5-4-1BB-Eva1CAR-T, shGFP-4-1BB-Eva1CAR-T, shCUL5-CD79A/40-Eva1CAR-T, shGFP-CD79A/40-Eva1CAR-T) were intravenously administered at 0.5x10e5/mouse. Thereafter, luciferin was administered every certain period, luminescence of tumor cells was photographed, and the tumor volume was measured. When the tumor volume exceeded 2000mm3, the mouse was subjected to euthanasia treatment.

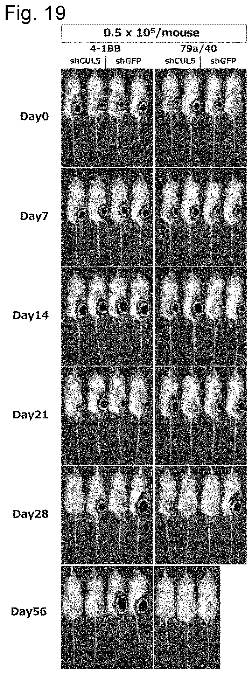

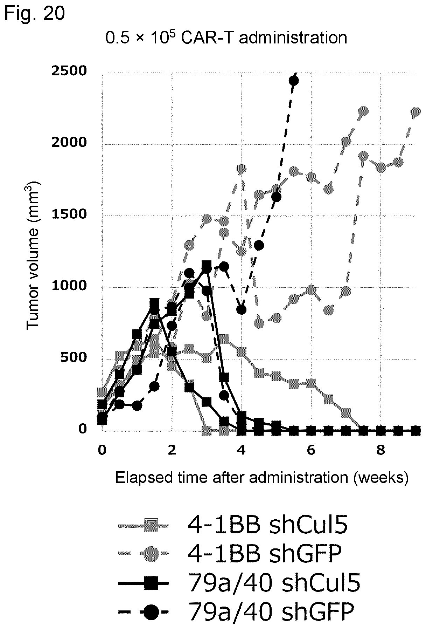

The photographed images of luminescence are shown in Fig. 19, and the measurement results of the tumor volume are shown in Fig. 20.

## Claims

1. A polynucleotide comprising an expression cassette of a CUL5 gene expression suppressing polynucleotide and an expression cassette of a chimeric antigen receptor.

2. The polynucleotide according to claim 1, wherein the CUL5 gene expression suppressing polynucleotide is at least one selected from the group consisting of CUL5-specific siRNA, CUL5-specific miRNA, and CUL5-specific antisense polynucleotide.

3. The polynucleotide according to claim 1, wherein the CUL5 gene expression suppressing polynucleotide is CUL5-specific siRNA.

4. The polynucleotide according to claim 1, wherein the polynucleotide is a vector.

5. The polynucleotide according to claim 1, wherein the polynucleotide is a viral vector or a viral genome.

6. The polynucleotide according to claim 1, wherein a target antigen of the chimeric antigen receptor is a cancer antigen.

7. A cell comprising the polynucleotide according to any of claims 1 to 6.

8. The cell according to claim 7, wherein expression of CUL5 gene is reduced by the CUL5 gene expression suppressing polynucleotide, and the cell expresses a chimeric antigen receptor.

9. The cell according to claim 8, wherein the cell is a lymphocyte cell.

10. A pharmaceutical composition comprising the cell according to claim 7.

11. The pharmaceutical composition according to claim 10, which is for use in a treatment, prevention, or improvement of cancer.

12. A cell that is modified to reduce expression of CUL5 gene and expresses a chimeric antigen receptor.
